# EUROPÄISCHE PATENTSCHRIFT

(11) **EP 1 372 871 B1**
(45) Veröffentlichungstag und Bekanntmachung des Hinweises auf die Patenterteilung: **12.07.2006**
(21) Anmeldenummer: 02735180.8
(22) Anmeldetag: 26.03.2002
(51) Int. Cl.: B05D 5/08, A61C 1/00, A61C 1/16, A61B 19/00

(54) **BESCHICHTUNG FÜR HANDSTÜCK**
COATING FOR A HANDLE
REVETEMENT POUR PIECE A MAIN

(30) Priorität: 26.03.2001 DE 10114657
(43) Veröffentlichungstag der Anmeldung: 02.01.2004
(73) Patentinhaber: Kaltenbach & Voigt GmbH, 88400 Biberach / Riss (DE)
(72) Erfinder: KAYSER, Oliver, 53797 Lohmar/Neuhonrath (DE); EIBOFNER, Eugen, 88400 Biberach (DE); LINGENHÖLE, Bernhard, 88447 Warthausen (DE)
(74) Vertreter: Schmidt-Evers, Jürgen
(86) Internationale Anmeldenummer: PCT/EP2002/003392
(87) Internationale Veröffentlichungsnummer: WO 2002/076633

(56) Entgegenhaltungen:
- WO-A-89/01534
- DD-A- 153 976
- DE-A- 2 146 906
- FR-A- 2 622 600
- US-A- 5 545 439
- PATENT ABSTRACTS OF JAPAN vol. 013, no. 286 (C-613), 29. Juni 1989 (1989-06-29) & JP 01 083252 A (KAIJIRUSHI HAMONO KAIHATSU CENTER:KK), 29. März 1989 (1989-03-29)

## Beschreibung

Die vorliegende Erfindung betrifft eine Beschichtung für ein Handstück für medizinische, zahnmedizinische oder chirurgische Zwecke oder ein Handstück mit dieser Beschichtung bzw. ein Verfahren zum Beschichten der Oberfläche eines derartigen Handstücks.

Den Oberflächeneigenschaften von zahnmedizinischen oder chirurgischen Handstücken kommt eine besondere Bedeutung zu. So war es seit Anbeginn der Handstück-Technik das Bestreben, die Oberflächen der Handstücke derart zu gestalten, daß sie einerseits aus hygienischen Gründen schmutzabweisend sind und andererseits ein Verrutschen in den Fingern eines Benutzers vermieden wird, insbesondere auch dann, wenn die Handstücke feucht sind.

Eine bekannte Lösung, mit der diesem Begehren Rechnung getragen wurde, bestand darin, die Oberfläche des Handstücks mit wabenartigen Facetten zu versehen. Hierdurch weißt die Handstückoberfläche ein Profil auf, welches einen gewissen Halt bietet und durch das dementsprechend die Handhabung verbessert wird. Allerdings kann mit dieser Lösung keine vollkommen schmutzabweisende Oberfläche realisiert werden, da sich an den Facetten nach wie vor Schmutz festsetzen kann.

Eine weitere bekannte Lösung bei einem zahnärztlichen Handstück bestand darin, daß im mittleren Griffbereich sogenannte Rändelringe vorgesehen wurden. Auch diese Rändelringe tendieren allerdings dazu, Schmutz aufzunehmen. In einer Weiterbildung wurde daher die Oberfläche im mittleren Bereich des Handstücks wellenartig gestaltet und gleichzeitig die Oberfläche aufgerauht.

Den oben genannten Beispielen ist zu entnehmen, daß bei der Ausgestaltung der Handstücke stets ein Kompromiß zwischen einer einerseits möglichst glatten und einer andererseits möglichst rauhen Oberfläche geschlossen werden muß. Je glatter die Oberfläche ist, desto geringer ist die Neigung, Schmutz aufzunehmen. Andererseits wird durch eine rauhe Oberfläche die Handhabung erleichtert und die Gefahr reduziert, daß das Handstück während der Anwendung verrutscht.

Es ist daher Aufgabe der vorliegenden Erfindung, eine weitere Möglichkeit für die Oberflächengestaltung eines Handstücks für zahnmedizinische oder chirurgische Zwecke anzugeben, durch welche einerseits die Tendenz dazu, Schmutz oder Flüssigkeit, insbesondere Wasser, aufzunehmen, reduziert wird und welche auf der anderen Seite eine optimale Handhabung des Handstücks ermöglicht.

Das Dokument DE 19505792 A1 offenbart ein Handstück mit einer Polytetrafluoroethylen-Beschichtung.

Die Aufgabe wird durch eine Beschichtung für ein Handstück, welche die Merkmale des Anspruches 1 aufweist, gelöst.

Die erfindungsgemäße Beschichtung besteht aus einer Hartstoffschicht, welche eine vorgegebene Rauheit aufweist, sowie aus einer auf die Hartstoffschicht aufgebrachten Kunststoffschicht mit einer reduzierten Benetzbarkeit. Die Hartstoffschicht dient zunächst dazu, die Handstückoberfläche vor äußeren Einwirkungen insbesondere mechanischer Art zu schützen. Durch ihre vorgegebene Rauheit wird eine sichere Handhabung des Handstücks auch im feuchten Zustand gewährleistet. Die auf die Hartstoffschicht aufgebrachte Kunststoffschicht verleiht der Beschichtung schmutzabweisende Eigenschaften. Im Vergleich zu den durch die Rauheit der Hartstoffschicht bedingten Oberflächenverformungen ist die Dicke der Kunststoffschicht allerdings nur sehr gering, so daß die durch die Hartstoffschicht ursprünglich erzielte Rauheit durch die zusätzliche Beschichtung lediglich unwesentlich reduziert wird. Vorzugsweise weist die Hartstoffschicht eine gemittelte Rauhtiefe Rz von ca. 2-12 µm - insbesondere von 4-8 µm - sowie einen arithmetischen Mittelrauhwert Ra von ca. 0,2-1,0 µm auf. Idealerweise werden Rauhwerte von Rz ca. 5-6 µm und Ra ca. 02,-0,5 µm erreicht. Dabei besteht die Hartstoffschicht vorzugsweise aus Chrom-Nitrid (CrN). Die Kunstoffschicht kann beispielsweise durch einen fluorhaltigen Kunststoff, insbesondere durch Polytetrafluorethylen-(PTFE) - auch bekannt unter dem Namen Teflon - gebildet werden.

Die Benetzbarkeit der Kunststoffschicht ist bezüglich der Benetzbarkeit der glatten Oberfläche eines Handstücks aus Stahl reduziert. Es sind zwar Handstücke aus Kunststoff bekannt geworden, jedoch wurde dazu Polyetheretherketon(PEEK) verwendet. Dieser Kunststoff weist keine reduzierte Benetzbarkeit auf. Für eine Kunststoffschicht mit reduzierter Benetzbarkeit eignen sich nicht polare Kunststoffe. Die Benetzbarkeit der Kunststoffschicht aus einem nicht polaren Kunststoff ist bezüglich der Benetzbarkeit von Stahl und Polyetheretherketon (PEEK) wesentlich reduziert.

Gemäß einem weiteren Aspekt der vorliegenden Erfindung wird ein Verfahren zum Beschichten der Oberfläche eines Griffbereichs eines zahnärztlichen oder chirurgischen Handstücks angegeben, welches aus mehreren Schritten besteht. In einem ersten Schritt wird eine Hartstoffschicht mit einer vorgegebenen Rauheit auf die Handstückoberfläche aufgebracht und in einem darauffolgenden Schritt die Hartstoffschicht mit einer Kunststoffschicht mit einer reduzierten Benetzbarkeit versiegelt.

Das Aufbringen der Hartstoffschicht erfolgt vorzugsweise durch ein Vakuumbeschichtungsverfahren, insbesondere durch ein sogenanntes PVD-(Physical Vapor Deposition - physikalische Abscheidung aus der Gasphase) Verfahren. Hierfür wird das Substrat innerhalb einer Vakuumkammer angeordnet. Zum Aufbringen einer Chrom-Nitrid-Schicht beispielsweise wird dann innerhalb der Kammer Chrom verdampft und gleichzeitig Stickstoff in einer geeigneten Menge in die Kammer eingeleitet. Das Aufbringen erfolgt vorzugsweise bei einem im Vergleich zu üblichen PVD-Beschichtungsverfahren erhöhtem Totaldruck, bei dem die gewünschte Rauheit für die Hartstoffschicht erzielt wird, beispielsweise bei einem Druck von ca. 0,8-5,0 x 10⁻² mbar.

Gemäß einer vorteilhaften Weiterbildung der vorliegenden Erfindung wird in einer letzten Phase des Aufbringens der Hartstoffschicht der in die Kammer eingeleitete Stickstoff zu einem gewissen Teil durch ein Inertgas, beispielsweise durch Argon oder ein anderes Edelgas ersetzt. Hierdurch weisen die oberen Bereiche der Hartstoffschicht eine leicht veränderte chemische Zusammensetzung auf, was zur Folge hat, daß ein bestimmter, gräulich-blauer Farbton erzielt wird. Vorzugsweise erfolgt das teilweise Ersetzen des Stickstoffs durch das Inertgas während des Aufbringens der gesamten oder der letzten 400 nm der Hartstoffschicht.

Das Versiegeln der Hartstoffschicht mit der Kunststoffschicht erfolgt vorzugsweise durch eine naß-chemische Reaktion. Nach dem Aufbringen der Kunststoffschicht wird diese bei etwa 260°C eingebrannt.

Die erfindungsgemäße Beschichtung stellt einen optimalen Kompromiß zwischen einer möglichst rauhen Oberfläche, welche eine gute Handhabung des Handstücks gewährleistet, sowie einer möglichst glatten und damit schmutzabweisenden Oberfläche dar. Darüber hinaus wird ein optimaler Schutz des Handstücks vor äußeren Einflüssen, mechanischer aber auch chemischer Art erzielt.

Die Erfindung bezieht sich auch auf ein medizinisches oder zahnmedizinisches oder chirurgisches Handstück mit der erfindungsgemäßen Beschichtung. Die vorbeschriebenen Vorteile gelten auch für ein solches erfindungsgemäßes Handstück. Dabei kann das Handstück sich gerade oder bogenförmig gekrümmt erstrecken oder abgewinkelt sein, wie es an sich üblich ist. Die erfindungsgemäße Beschichtung und das erfindungsgemäße Verfahren zum Beschichten eignen sich für diese Formen des Handstücks sehr gut.

Im folgenden soll die Erfindung anhand der beiliegenden Zeichnung näher erläutert werden. Es zeigen:
- Fig. 1: ein zahnmedizinisches Handstück in allgemeiner Form,
- Fig. 2: ein Handstück in abgewandelter Form, und
- Fig. 3: eine vergrößerte Darstellung der erfindungsgemäßen Beschichtung im Schnitt.

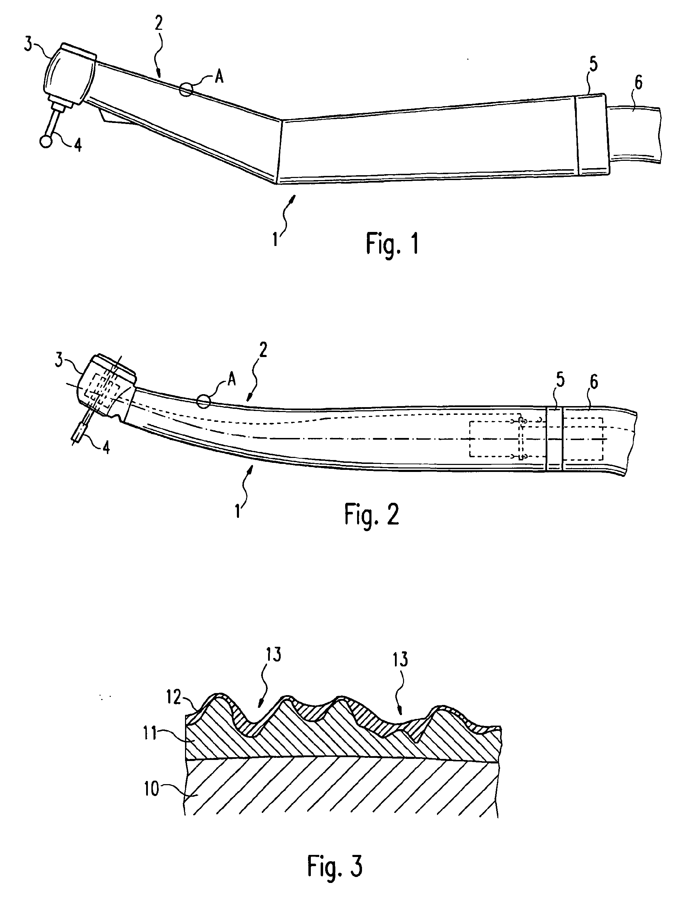

Bei dem in den Figuren 1 und 2 dargestellten Handstück 1 handelt es sich um ein zahnärztliches Handstück, z.B. ein Bohrhandstück. Selbstverständlich kann die erfindungsgemäße Beschichtung aber auch bei sämtlichen anderen zahnärztlichen oder chirurgischen Handstücken zum Einsatz kommen. Das dargestellte Handstück 1 besteht aus einer länglichen Griffhülse, die leicht abgewinkelt ist (Fig. 1) oder bogenförmig gekrümmt sein kann (Fig. 3). Am vorderen Ende befindet sich ein Kopfgehäuse 3, innerhalb dessen eine nicht dargestellte Antriebsvorrichtung zum Antrieb eines zahnärztlichen Werkzeugs - im vorliegenden Beispiel zum Antrieb eines Bohrers 4 - angeordnet ist. An seinem hinteren Ende ist das Handstück 1 über ein Kupplungsstück 5 drehbar mit einem Versorgungsschlauch 6 zur Zuführung von Versorgungs- und Behandlungsmedien, Licht oder elektrischer Energie verbunden. Das Werkzeug ist in eine Haltevorrichtung einspannbar, die am vorderen Ende des Handstücks 1 angeordnet ist.

Zur Anwendung wird das Handstück 1 von einem Benutzer an seinem vorderen Griffbereich 2 gehalten. Zumindest dieser vordere Griffbereich 2 weist die erfindungsgemäße Beschichtung auf. Es wäre allerdings auch denkbar, daß die gesamte Oberfläche des Handstücks 1 mit dieser Beschichtung versehen ist.

Bevor die Herstellung der erfindungsgemäßen Beschichtung beschrieben wird, soll zunächst anhand von Figur 3 deren struktureller Aufbau erläutert werden. Figur 3 zeigt dabei einen Ausschnitt A der Handstückoberfläche in vergrößerter Darstellung im Schnitt. Mit dem Bezugszeichen 10 ist dabei die Oberfläche des Handstücks selbst bezeichnet, die erfindungsgemäße Beschichtung besteht aus den beiden darüber angeordneten Schichten 11 und 12.

Die Oberflächenstruktur der erfindungsgemäßen Beschichtung wird durch die auf die Handstückoberfläche 10 aufgebrachte Hartstoffschicht 11 bestimmt, bei der es sich im vorliegenden Fall um eine Chrom-Nitrid-(CrN) Schicht handelt. Dieses Material findet aufgrund seiner extrem hohen Härte häufig in der Druck- und Prägetechnik Verwendung oder dient zum Schutz für korrosiv beanspruchte Werkzeuge und als dekorative Schicht.

Im Vergleich zu üblichen Chrom-Nitrid-Beschichtungen oder auch anderen Hartstoffschichten weist die dargestellte Hartstoffschicht 11 erfindungsgemäß jedoch eine deutlich höhere Rauheit auf. Zur Charakterisierung der Rauheit einer Oberfläche werden üblicherweise zwei Größen bestimmt, zum einen die gemittelte Rauhtiefe Rz und zum anderen der arithmetische Mittenrauhwert Ra. Dabei handelt es sich bei der gemittelten Rauhtiefe Rz um das arithmetische Mittel aus den größten Einzelrauhtiefen mehrerer aneinandergrenzender Einzelmeßstrecken. Der weiter verbreitete arithmetische Mittenrauhwert Ra hingegen ist der arithmetische Mittelwert der absoluten Werte der Profilabweichungen innerhalb einer vorgegebenen Bezugsstrecke. Dieser Wert ist grundsätzlich kleiner als der auf einem gleichen Rauheitsprofil ermittelte Wert für die gemittelte Rauhtiefe Rz. Die in Figur 3 dargestellte Hartstoffschicht 11 weist vorzugsweise eine gemittelte Rauhtiefe Rz von etwa 2-12 µm - besonders bevorzugt etwa 4-8 µm und idealerweise von 5-6 µm - sowie einen arithmetischen Mittenrauhwert Ra von etwa 0,2-1,0 µm bzw. vorzugsweise von 0,2-0,5 µm auf. Es handelt sich somit nicht um eine glatte sondern um eine mit einer Vielzahl von Poren 13 versehene Struktur, die eine besonders gute Handhabung des Handstücks gewährleistet, selbst bei der Anwesenheit von Feuchtigkeit.

Auf die Oberseite der Hartstoffschicht 11 ist die Kunststoff-Versiegelung 12 aufgebracht, welche der erfindungsgemäßen Beschichtung die zweite angestrebte Eigenschaft verleiht, nämlich möglichst schmutzabweisend oder flüssigkeitsabweisend oder wasserabweisend zu sein. Die Kunststoffschicht 12, bei der es sich vorzugsweise um eine Polytetrafluorethylen-Schicht oder einen anderen fluorhaltigen Kunststoff handelt, ist im Vergleich zu den durch die Poren 13 der Hartstoffschicht 11 vorgegebenen Oberflächenverformungen nur sehr dünn und gleicht damit die Rauheit der Hartstoffschicht 11 zu einem nur sehr geringen Grad aus, so daß die rutschfeste Handhabung des Handstücks nicht beeinträchtigt wird. Sowohl die Hartstoffschicht 11 als auch die Kunststoffschicht 12 tragen ferner dazu bei, das Handstück vor äußeren Einflüssen zu schützen. Die Hartstoffschicht 11 dient dabei insbesondere dem mechanischen Schutz des Handstücks, während die Kunststoff-Versiegelung 12 chemische Einflüsse reduziert.

Im folgenden soll das erfindungsgemäße Verfahren zum Aufbringen der Beschichtung erläutert werden. Die untere Hartstoffschicht wird dabei durch ein Vakuumbeschichtungsverfahren, im vorliegenden Fall durch ein sogenanntes PVD-(Physical Vapor Deposition) Verfahren aufgebracht. Dabei werden metallische Ausgangsmaterialien durch Eintrag thermischer Energie aus einer festen Phase in die Gasphase überführt, ionisiert und auf das Substrat hin beschleunigt. Der Beschichtungsprozeß läuft dabei in einer evakuierten Vakuumkammer ab, wobei durch die kontrollierte Zugabe weiterer Komponenten in Form von Reaktivgasen die gewünschte Hartstoffschicht erhalten werden.

Die Verfahren sowie Vorrichtungen zum Durchführen solcher Vakuumbeschichtungen sind bereits hinlänglich bekannt, beispielsweise aus der DE 41 25 365 C1. Im folgenden soll daher das Verfahren lediglich zusammengefaßt und auf die Besonderheiten zum Erzielen der gewünschten Rauheit eingegangen werden.

Die Beschichtung erfolgt vorzugsweise nach einem sogenannten Lichtbogenverdampfen (arc-coating) bei einem reduzierten Totaldruck in einer gasdichten Vakuumkammer. Der Abtrag erfolgt dabei von metallischen Spenderronden durch oberflächengebundene Lichtbögen. Diese Spenderronden werden zuvor elektrisch isolierend in die Vakuumkammer montiert und mit ca. 30 Volt negativ gegen das Potential der Kammerwand vorgespannt. Aufgrund der hohen Energie der Lichtbögen wird das in fester Form vorliegende Spendermaterial lokal verdampft und ionisiert. Über eine negative Spannung werden dann die Ionen zu dem Substrat hin beschleunigt.

Zum Aufbringen der Chrom-Nitrid-Schicht wird reines Chrom als Spendermaterial verwendet. Durch zusätzliches Einleiten von reinem Stickstoff in die Vakuumkammer werden die Chrom-Ionen auf der zu beschichtenden Handstückoberfläche zu Chrom-Nitrid CrN ergänzt. Mit Hilfe eines Massenflussreglers in Abstimmung mit der Saugleistung der Vakuumpumpen kann dabei die Menge des eingegebenen Stickstoffs derart eingestellt werden, daß sich in der Beschichtungskammer ein Totaldruck von ca. 0,8-5,0 x 10⁻² mbar ergibt.

Der oben angegebene Druckwert ist höher als der üblicherweise beim Aufdampfen von Hartstoffschichten vorliegende Druck. Hierdurch wird die gewünschte Oberflächenstruktur mit einer deutlich höheren Rauheit als üblich erzielt. Um der auf das Handstück aufgedampften Hartstoffschicht eine besondere Farbe zu verleihen, wird dabei während des Aufbringens der gesamten oder der letzten 400nm die in der Vakuumkammer vorliegende Stickstoffatmosphäre durch Zugabe von Argon verdünnt. Hierdurch weisen die oberen Bereiche der Hartstoffschicht eine leicht veränderte chemische Zusammensetzung auf, wodurch typischerweise ein gräulich-blauer Farbton entsteht. Anstelle von Argon kann allerdings auch ein anderes Edelgas oder Inertgas verwendet werden.

Der Vorteil des angegebenen PVD-ARC-Verfahrens liegt darin, daß Schichten schon bei einer relativ niedrigen Beschichtungstemperatur von weniger als 200°C abgeschieden werden können. Vorzugsweise wird dabei im Vorfeld der Beschichtung die Substratoberfläche durch einen Plasmareinigungsprozeß zusätzlich gesäubert.

Das anschließende Aufbringen der Teflon-Schicht erfolgt durch ein naß-chemisches Verfahren an Luftatmosphäre. Anschließend wird die Versiegelung bei ca. 260°C eingebrannt. Alternativ dazu kann die Kunststoff-Versiegelung allerdings auch mittels Plasmapolymerisation, d.h. mit einer Dünnschichttechnik aufgebracht werden.

Das angegebene Verfahren stützt sich somit auf die bereits vorliegenden grundlegenden Erkenntnisse bei der Beschichtung von Werkstücken und unterscheidet sich von den bekannten PVD-Verfahren in erster Linie dadurch, daß durch die Wahl eines höheren Druckwerts die gewünschte Rauheit erzielt und zum anderen durch den teilweisen Ersatz des Stickstoffs durch Argon der Hartstoffschicht eine bestimmte Farbe verliehen wird. Gleichzeitig verleiht die erfindungsgemäße Beschichtung dem Handstück hervorragende Eigenschaften, durch die zum einen die Handhabung verbessert und zum anderen die Tendenz, Schmutz aufzunehmen, reduziert wird. Diese sich eigentlich entgegenstehenden Eigenschaften werden insbesondere durch die erfindungsgemäße Kombination einer rauhen Hartstoffschicht mit einer schmutzabweisenden Kunststoff-Versiegelung erreicht.

## Patentansprüche

1. Medizinisches oder zahnmedizinisches Handstück (1), das eine rauhe Oberflächenschicht aufweist,
wobei die Oberflächenschicht aus einer rauhen Hartstoffschicht (11) und einer auf dieser aufgebrachten Kunststoffschicht (12) reduzierter Benetzbarkeit besteht, die so dünn ist, dass sie die Rauheit der Hartstoffschicht (11) nur zu einem geringen Grad ausgleicht.

2. Handstück nach Anspruch 1,
**dadurch gekennzeichnet,**
**dass** die Hartstoffschicht (11) aus Chrom-Nitrid (CrN) besteht.

3. Handstück nach Anspruch 1 oder 2,
**dadurch gekennzeichnet,**
**dass** die Hartstoffschicht (11) im wesentlichen eine Rauheit mit Rz = 2-12 µm und Ra = 0,2 bis 1,0 µm aufweist.

4. Handstück nach Anspruch 3,
**dadurch gekennzeichnet,**
**dass** die Hartstoffschicht (11) im wesentlichen eine Rauheit mit Rz = 4-8 µm und Ra = 0,2 bis 1,0 µm aufweist.

5. Handstück nach Anspruch 4,
**dadurch gekennzeichnet,**
**dass** die Hartstoffschicht (11) im wesentlichen eine Rauheit mit Rz = 5-6 µm und Ra = 0,2 bis 0,5 µm aufweist.

6. Handstück nach einem der vorherigen Ansprüche,
**dadurch gekennzeichnet,**
**dass** die Kunststoffschicht (12) durch einen fluorhaltigen Kunststoff gebildet wird.

7. Handstück nach Anspruch 6,
**dadurch gekennzeichnet,**
**dass** die Kunststoffschicht (12) eine Polytetrafluorethylen-Schicht ist.

8. Handstück nach einem der vorherigen Ansprüche,
**dadurch gekennzeichnet,**
**dass** das Handstück (1) eine längliche Griffhülse mit einem Griffbereich aufweist und an seinem hinteren Ende ein Kupplungsstück (5) aufweist, mit dem es mit einem Versorgungsschlauch (6) verbindbar ist, und an seinem vorderen Ende eine Haltevorrichtung für ein Werkzeug aufweist,
wobei die rauhe Oberflächenschicht wenigstens im Griffbereich angeordnet ist.

9. Verfahren zum Beschichten der Oberfläche eines Griffbereichs (2) eines Handstücks (1) für zahnmedizinische oder chirurgische Zwecke mit folgenden Schritten:
a) Aufbringen einer Hartstoffschicht (11) mit einer vorgegebenen Rauheit auf die Handstückoberfläche und
b) Versiegeln der Hartstoffschicht (11) mit einer Kunststoffschicht (12) mit einer reduzierten Benetzbarkeit derart, dass die Kunststoffschicht (12) die Rauheit der Hartstoffschicht (11) nur zu einem geringen Teil ausgleicht.

10. Verfahren nach Anspruch 9,
**dadurch gekennzeichnet,**
**dass** die Hartstoffschicht (11) aus Chrom-Nitrid (CrN) besteht.

11. Verfahren nach Anspruch 9 oder 10,
**dadurch gekennzeichnet,**
**dass** in Schritt a) die Hartstoffschicht (11) durch ein Vakuumbeschichtungsverfahren aufgebracht wird.

12. Verfahren nach Anspruch 10 und 11,
**dadurch gekennzeichnet,**
**dass** zum Aufbringen der Hartstoffschicht (11) das Handstück (1) in einer Vakuumkammer angeordnet wird, wobei zum Beschichten Chrom verdampft und gleichzeitig Stickstoff in die Kammer eingeleitet wird.

13. Verfahren nach Anspruch 12,
**dadurch gekennzeichnet,**
**dass** das Beschichten bei einem Totaldruck von ca. 0,8-5,0·10⁻² mbar erfolgt.

14. Verfahren nach Anspruch 12 oder 13,
**dadurch gekennzeichnet,**
**dass** während einer letzten Phase der Handstück der in die Kammer eingeleitete Stickstoff teilweise durch ein Inertgas ersetzt wird.

15. Verfahren nach Anspruch 14,
**dadurch gekennzeichnet,**
**dass** als Inertgas Argon verwendet wird.

16. Verfahren nach Anspruch 14 oder 15,
**dadurch gekennzeichnet,**
**dass** das Ersetzen des Stickstoffs durch das Inertgas während des Aufdampfens der letzten 400nm der Hartstoffschicht (11) erfolgt.

17. Verfahren nach einem der Ansprüche 9 bis 16,
**dadurch gekennzeichnet,**
**dass** die Hartstoffschicht (11) im wesentlichen eine Rauheit mit Rz = 2-12 µm und Ra = 0,2-1,0 µm aufweist.

18. Verfahren nach Anspruch 17,
**dadurch gekennzeichnet,**
**dass** die Hartstoffschicht (11) im wesentlichen eine Rauheit mit Rz = 4-8 µm und Ra = 0,2-1,0 µm aufweist.

19. Verfahren nach Anspruch 18,
**dadurch gekennzeichnet,**
**dass** die Hartstoffschicht (11) im wesentlichen eine Rauheit mit Rz = 5-6 µm und Ra = 0,2-0,5 µm aufweist.

20. Verfahren nach einem der Ansprüche 9 bis 19,
**dadurch gekennzeichnet,**
**dass** in Schritt b) die Kunststoffschicht (12) durch eine naß-chemische Reaktion aufgebracht wird.

21. Verfahren nach einem der Ansprüche 9 bis 19,
**dadurch gekennzeichnet,**
**dass** in Schritt b) die Kunststoffschicht (12) mittels Plasmapolymerisation aufgebracht wird.

22. Verfahren nach Anspruch 20 oder 21,
**dadurch gekennzeichnet,**
**dass** nach dem Aufbringen der Kunststoffschicht (12) diese bei etwa 260°C eingebrannt wird.

23. Verfahren nach einem der Ansprüche 9 bis 22,
**dadurch gekennzeichnet,**
**dass** in Schritt b) eine fluorhaltige Kunststoffschicht aufgebracht wird.

24. Verfahren nach Anspruch 23,
**dadurch gekennzeichnet,**
**dass** in Schritt b) eine Polytetrafluorethylen-Schicht aufgebracht wird.

## Claims

1. Medical or dental-medical handpiece (1), having a rough surface coating,
wherein the surface coating is of a rough hard material layer (11) and a plastic layer (12) of reduced wettability, applied to this hard material layer, which plastic layer is so thin that the roughness of the hard material layer (11) is compensated only to a small degree.

2. Hand piece according to claim 1,
**characterized in that**,
the hard material layer (11) is of chromium nitride (CrN).

3. Hand piece according to claim 1 or 2,
**characterized in that**,
the hard material layer (11) has in substance a roughness with Rz = 2-12 µm and Ra = 0.2 to 1.0 µm.

4. Hand piece according to claim 3,
**characterized in that**,
the hard material layer (11) has in substance a roughness with Rz = 4 - 8 µm and Ra = 0.2 to 1.0 µm.

5. Hand piece according to claim 4,
**characterized in that**,
the hard material layer (11) has in substance a roughness with Rz = 5 - 6 µm and Ra = 0.2 to 0.5 µm.

6. Hand piece according to any preceding claim,
**characterized in that**,
the plastic layer (12) is formed of a fluorine-containing plastic.

7. Hand piece according to claim 6,
**characterized in that**,
the plastic layer (12) is a polytetrafluoroethylene layer.

8. Handpiece according to any preceding claim,
**characterized in that**,
the handpiece (1) has an elongate grip sleeve with a grip region and a coupling piece (5) at its rearward end, with which coupling piece it can be connected with a supply hose (6), and at its forward end has a holder device for a tool,
wherein the rough surface coating is arranged at least in the grip region.

9. Method of coating the surface of a grip region (2) of a handpiece (1) for dental-medical or surgical purposes, having the following steps:
a) application of a hard material layer (11) having a predetermined roughness, on the handpiece surface and
b) sealing of the hard material layer (11) with a plastic layer (12) having a reduced wettability, such that the plastic layer (12) compensates the roughness of the hard material layer (11) only to a small part.

10. Method according to claim 9,
**characterized in that**,
the hard material layer (11) is of chromium nitride (CrN).

11. Method according to claim 9 or 10,
**characterized in that**,
in step a) the hard material layer (11) is applied by means of a vacuum coating process.

12. Method according to claims 10 and 11,
**characterized in that**,
for the application of the hard material layer (11) the handpiece (1) is arranged in a vacuum chamber, wherein for the coating chromium is evaporated and at the same time nitrogen is introduced into the chamber.

13. Method according to claim 12,
**characterized in that**,
the coating is effected at a total pressure of ca. 0.8 - 5.0 x 10⁻² mbar.

14. Method according to claim 12 or 13,
**characterized in that**,
during a final phase of the handpiece, the nitrogen introduced into the chamber is partially replaced with an inert gas.

15. Method according to claim 14,
**characterized in that**,
argon is employed as inert gas.

16. Method according to claim 14 or 15,
**characterized in that**,
the replacement of the nitrogen by the inert gas is effected during the vapor deposition of the last 400 nm of the hard material layer (11).

17. Method according to any of claims 9 to 16,
**characterized in that**,
the hard material layer (11) has in substance a roughness with Rz = 2 - 12 µm and Ra = 0.2 to 1.0 µm.

18. Method according to claim 17,
**characterized in that**,
the hard material layer (11) has in substance a roughness with Rz = 4 - 8 µm and Ra = 0.2 to 1.0 µm.

19. Method according to claim 18,
**characterized in that**,
the hard material layer (11) has in substance a roughness with Rz = 5 - 6 µm and Ra = 0.2 to 0.5 µm.

20. Method according to any of claims 9 to 19,
**characterized in that**,
in step b) the plastic layer (12) is applied by means of a wet chemical reaction.

21. Method according to any of claims 9 to 19,
**characterized in that**,
in step b) the plastic layer (12) is applied by means of plasma polymerization.

22. Method according to claim 20 or 21,
**characterized in that**,
after the application of the plastic layer (12) this is burned in at about 260°C.

23. Method according to any of claims 9 to 22,
**characterized in that**,
in step b) there is applied a fluorine-containing plastic layer.

24. Method according to claim 23,
**characterized in that**,
in step b) a polytetraflouroethylene layer is applied.

## Revendications

1. Manche d'instrument médical ou dentaire (1), qui présente une couche superficielle rugueuse,
selon lequel la couche superficielle se compose d'une couche de matière dure (11) rugueuse et d'une couche de plastique (12) de mouillabilité réduite, déposée sur la première, qui est si mince, qu'elle ne compense la rugosité de la couche de matière dure (11) qu'à un faible degré.

2. Manche selon la revendication 1, **caractérisé en ce que** la couche de matière dure (11) se compose de nitrure de chrome (CrN).

3. Manche selon la revendication 1 ou 2, **caractérisé en ce que** la couche de matière dure (11) présente sensiblement une rugosité avec Rz = 2 - 12 µm et Ra = 0,2 à 1,0 µm.

4. Manche selon la revendication 3, **caractérisé en ce que** la couche de matière dure (11) présente sensiblement une rugosité avec Rz = 4 - 8 µm et Ra = 0,2 à 1,0 µm.

5. Manche selon la revendication 4, **caractérisé en ce que** la couche de matière dure (11) présente sensiblement une rugosité avec Rz = 5 - 6 µm et Ra = 0,2 à 0,5 µm.

6. Manche selon l'une des revendications précédentes, **caractérisé en ce que** la couche de plastique (12) est formée d'un plastique fluoré.

7. Manche selon la revendication 6, **caractérisé en ce que** la couche de plastique (12) est une couche de polytétrafluoroéthylène.

8. Manche selon l'une des revendications précédentes, **caractérisé en ce que** le manche (1) présente une gaine de prise longitudinale dotée d'une zone de prise et, à son extrémité arrière, une pièce d'accouplement (5), avec laquelle il peut être raccordé à un tuyau d'alimentation (6), et, à son extrémité avant, un dispositif support pour un outil, la couche superficielle rugueuse étant disposée au moins dans la zone de prise.

9. Procédé de revêtement de la surface d'une zone de prise (2) d'un manche d'instrument (1) destiné à des fins dentaires ou chirurgicales, comprenant les étapes suivantes :
a) dépôt d'une couche de matière dure (11) ayant une rugosité prédéterminée sur la surface du manche et
b) comblement de la couche de matière dure (11) avec une couche de plastique (12) ayant une mouillabilité réduite, de sorte que la couche de plastique (12) compense la rugosité de la couche de matière dure (11) seulement en faible partie.

10. Procédé selon la revendication 9, **caractérisé en ce que** la couche de matière dure (11) se compose de nitrure de chrome (CrN).

11. Procédé selon la revendication 9 ou 10, **caractérisé en ce que** la couche de matière dure (11) est déposée à l'étape a) par un procédé de revêtement sous vide.

12. Procédé selon la revendication 10 et 11, **caractérisé en ce que**, pour le dépôt de la couche de matière dure (11), le manche d'instrument (1) est disposé dans une chambre sous vide, où, pour l'enduction, du chrome est vaporisé et de l'azote est simultanément introduit dans la chambre.

13. Procédé selon la revendication 12, **caractérisé en ce que** le revêtement s'effectue à une pression totale d'environ 0,8 à 5,0.10⁻² mbars.

14. Procédé selon la revendication 12 ou 13, **caractérisé en ce que**, pendant une dernière phase de revêtement du manche, l'azote introduit dans la chambre est partiellement remplacé par un gaz inerte.

15. Procédé selon la revendication 14, **caractérisé en ce que** l'on utilise de l'argon à titre de gaz inerte.

16. Procédé selon la revendication 14 ou 15, **caractérisé en ce que** le remplacement de l'azote par le gaz inerte s'effectue pendant la vaporisation des derniers 400 nm de la couche de matière dure (11).

17. Procédé selon l'une des revendications 9 à 16, **caractérisé en ce que** la couche de matière dure (11) présente sensiblement une rugosité avec Rz = 2 - 12 µm et Ra = 0,2 à 1,0 µm.

18. Procédé selon la revendication 17, **caractérisé en ce que** la couche de matière dure (11) présente sensiblement une rugosité avec Rz = 4 - 8 µm et Ra = 0,2 à 1,0 µm.

19. Procédé selon la revendication 18, **caractérisé en ce que** la couche de matière dure (11) présente sensiblement une rugosité avec Rz = 5 - 6 µm et Ra = 0,2 à 0,5 µm.

20. Procédé selon l'une des revendications 9 à 19, **caractérisé en ce que** la couche de plastique (12) est déposée à l'étape b) par une réaction chimique humide.

21. Procédé selon l'une des revendications 9 à 19, **caractérisé en ce que** la couche de plastique (12) est déposée à l'étape b) par polymérisation plasma.

22. Procédé selon la revendication 20 ou 21, **caractérisé en ce que**, après le dépôt de la couche de plastique (12), celle-ci est cuite à environ 260°C.

23. Procédé selon l'une des revendications 9 à 22, **caractérisé en ce qu'**une couche de plastique fluorée est déposée à l'étape b).

24. Procédé selon la revendication 23, **caractérisé en ce qu'**une couche de polytétrafluoroéthylène est déposée à l'étape b).
